(19) Europäisches Patentamt
European Patent Office
Office européen des brevets

(11) Numéro de publication : **0 314 575 B1**

(12) # FASCICULE DE BREVET EUROPEEN

(45) Date de publication du fascicule du brevet : **15.04.92 Bulletin 92/16**

(51) Int. Cl.$^5$ : **C07C 403/00**

(21) Numéro de dépôt : **88402710.3**

(22) Date de dépôt : **27.10.88**

(54) **Procédé de préparation du rétinal.**

(30) Priorité : **29.10.87 FR 8714980**

(43) Date de publication de la demande : **03.05.89 Bulletin 89/18**

(45) Mention de la délivrance du brevet : **15.04.92 Bulletin 92/16**

(84) Etats contractants désignés : **AT BE CH DE ES FR GB GR IT LI LU NL SE**

(56) Documents cités :
**JOURNAL OF THE AMERICAN CHEMICAL SOCIETY, vol. 102, no. 2, 1980, page 869; E. E. VAN TAMELEN et al, "Stereoselective Generation of a Nonaromatic, 3,5-Dioxygenated Steroidal System through Tricyclization of a Polyene Oxide"**

(56) Documents cités :
**PATENT ABSTRACTS OF JAPAN, vol. 9, no. 32 (C-265)(1755), 9 fevrier 1985; & JP-A-59 175 441**
**PATENT ABSTRACTS OF JAPAN, vol. 10, no. 355 (C-388)(2411), 29 novembre 1986; & JP-A-61 155 365**

(73) Titulaire : **Etablissement Public dit: CENTRE NATIONAL DE LA RECHERCHE SCIENTIFIQUE (CNRS)**
**15, Quai Anatole France**
**F-75007 Paris (FR)**

(72) Inventeur : **Julia, Marc**
**57 Rue Geoffroy Saint-Hilaire**
**F-75005 Paris (FR)**

(74) Mandataire : **Le Pennec, Magali et al**
**RHONE-POULENC SANTE, Service Brevets,**
**20 Avenue Raymond Aron**
**F-92165 Antony Cédex (FR)**

**EP 0 314 575 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne un nouveau procédé de préparation du rétinal de formule :

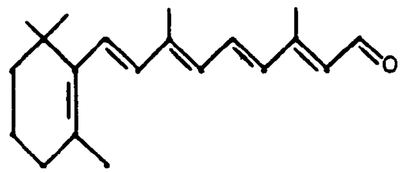

(I)

qui est un intermédiaire de synthèse de la vitamine A éventuellement sous forme d'acétate.

Il est connu (FR-A-2 171 497) de préparer le rétinal par condensation d'une sulfone $C_{15}$ de formule générale :

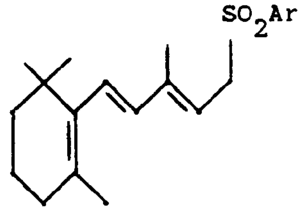

(II)

dans laquelle Ar représente un radical phényle éventuellement substitué, sur un halogénoacétal $C_5$ de formule générale :

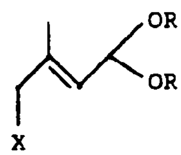

(III)

dans laquelle X représente un atome d'halogène (chlore, brome) et R représente un radical alcoyle contenant 1 à 4 atomes de carbone (de préférence, un radical méthyle ou éthyle), suivie de l'hydrolyse et de la désulfonation de la sulfone acétal $C_{20}$ de formule générale :

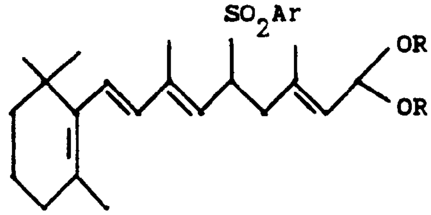

(IV)

dans laquelle Ar et R sont définis comme précédemment. Cependant, si ce procédé donne des résultats satisfaisants, il nécessite pour sa mise en oeuvre, en particulier pour la préparation de la sulfone $C_{15}$ de formule générale (II), l'utilisation d'un arylsulfinate dont la préparation et la récupération font appel à des techniques longues et coûteuses.

Il a maintenant été trouvé, et c'est ce qui fait l'objet de la présente invention, que le rétinal, contenant une proportion d'isomère tout trans généralement supérieure à 70 %, peut être obtenu avec de bons rendements par condensation d'un sulfure $C_{15}$ de formule générale :

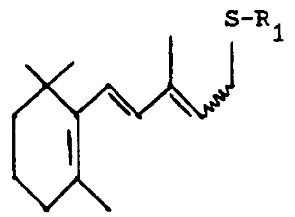

(V)

dans laquelle $R_1$ représente un radical aryle, de préférence un radical phényle éventuellement substitué, aralkyle, de préférence un radical benzyle, pyridyle, thiazolyl-2, benzothiazolyl-2, benzimidazolyl-2 ou benzoxazolyl-2, sur un halogénoacétal de formule générale (III), suivie de l'hydrolyse et de la désulfuration du sulfure acétal $C_{20}$ de formule générale :

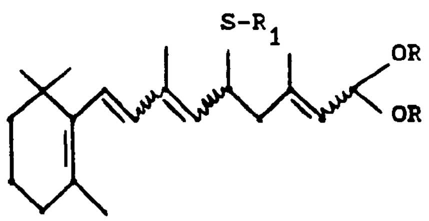

(VI)

dans laquelle $R_1$ et R sont définis comme précédemment en passant intermédiairement par le sulfure aldéhyde $C_{20}$ de formule générale :

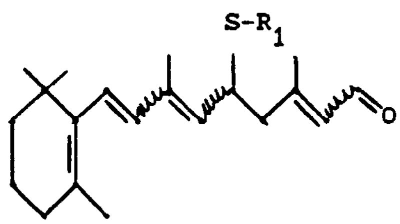

(VII)

dans laquelle $R_1$ est défini comme précédemment.

La condensation du sulfure $C_{15}$ de formule générale (V) sur l'halogénoacétal de formule générale (III) peut généralement être effectuée en présence d'un agent basique nécessaire pour réaliser l'anionisation du sulfure. Les agents basiques qui conviennent particulièrement bien peuvent être choisis parmi les hydroxydes, alcoolates, hydrures ou amidures des métaux alcalins, ou les composés organométalliques tels que les organozinciques, les organolithiens ou les organomagnésiens. Ils peuvent être utilisés seuls ou en association avec un autre agent basique destiné à neutraliser l'hydracide formé.

Généralement la réaction est effectuée à une température comprise entre -100 et 100°C.

La réaction peut être mise en oeuvre dans un solvant organique choisi parmi les hydrocarbures aliphatiques (hexane), les hydrocarbures aromatiques (benzène, toluène), les alcools (méthanol, éthanol), les éthers (éther diéthylique, dioxanne, tétrahydrofuranne), les amides (diméthylformamide, diméthylacétamide), le diméthylsulfoxyde, la N-méthylpyrrolidone ou l'hexaméthylphosphotriamide.

Par exemple la condensation peut être effectuée en présence de butyllithium et de diazabicyclooctane en opérant dans le tétrahydrofuranne à une température voisine de -30°C.

Le sulfure acétal de formule générale (VI) peut être transformé en sulfure aldéhyde de formule générale (VII) par hydrolyse en milieu acide. Par exemple l'hydrolyse peut être réalisée en présence d'acide oxalique à une température voisine de 20°C.

La désulfuration du sulfure aldéhyde de formule générale (VII) peut être effectuée par traitement au moyen d'un agent basique minéral ou organique tel qu'un hydroxyde alcalin, un carbonate alcalin ou un alcoolate alcalin. Par exemple, la désulfuration peut être réalisée à une température voisine de 20°C en utilisant le méthylate de sodium dans le méthanol ou l'éthylate de sodium dans l'éthanol.

Le procédé selon l'invention permet d'obtenir le rétinal à partir du sulfure $C_{15}$ avec des rendements généralement supérieurs à 75 %.

Selon la stéréoisomérie du sulfure $C_{15}$ de formule générale (V) et de l'halogénoacétal de formule générale (III), le sulfure acétal $C_{20}$ de formule générale (VI), de même que le sulfure aldéhyde $C_{20}$ de formule générale (VII) est le plus souvent obtenu sous forme d'un mélange de stéréoisomères. Quelle que soit la composition du mélange, le procédé selon l'invention permet d'obtenir un mélange de rétinals dans lequel l'isomère tout trans représente 70 %.

Le rétinal obtenu selon le procédé de la présente invention peut être transformé en vitamine A par application des méthodes connues.

Le sulfures $C_{15}$ de formule générale (V) peuvent être obtenus par action d'un thiol sur le vinyl β-ionol en présence d'une quantité catalytique d'un acide fort. Par exemple, le sulfure $C_{15}$ de formule générale (V) dans laquelle $R_1$ représente le radical phényle peut être obtenu par action du thiophénol sur le vinyl β-ionol en présence d'acide fluorosulfonique en opérant dans le nitrométhane à une température voisine de 0°C.

L'exemple suivant, donné à titre non limitatif, montre comment l'invention peut être mise en pratique.

EXEMPLE

1) Dans 40 cm3 de tétrahydrofuranne anhydre, on dissout, sous atmosphère d'argon, 4,5 g de phénylsulfure $C_{15}$ (E/Z = 70/30) (13,6 mmoles) et 1,84 g de diazabicyclooctane (16,3 mmoles).

On ajoute goutte à goutte, en 15 minutes, 11 cm3 d'une solution 1,48N de n.butyllithium dans l'hexane puis, en 10 minutes, 3,10 g de chloroacétal diméthylique $C_5$ (E/Z = 75/25) titrant 87 % (16 mmoles) en maintenant la température à -30°C.

Le mélange réactionnel rouge sombre se décolore progressivement et devient jaune orangé. On agite pendant 1 heure à -30°C puis laisse remonter la température au voisinage de 20°C. On ajoute alors 1 g de chlorure d'ammonium et laisse reposer pendant une nuit à une température comprise entre 18 et 20°C.

Après évaporation du tétrahydrofuranne à 20°C sous pression réduite, le résidu huileux est repris par de l'éther (30 cm3) puis on ajoute un mélange acide acétique-eau (50-50 en volumes) jusqu'à pH voisin de 7 puis immédiatement une solution aqueuse saturée de bicarbonate de sodium (30 cm3) et une solution saturée de chlorure de sodium (30 cm3).

Après décantation, les phases organiques sont séchées sur sulfate de sodium. Après filtration et évaporation du solvant sous pression réduite on obtient 6,20 g d'une huile jaune pâle qui est purifiée par chromatographie sur alumine neutre activée en éluant avec 1 litre de pentane puis 1 litre de pentane à 1 % d'éther éthylique puis par 2 litres de pentane à 2 % d'éther éthylique.

On recueille ainsi 1,2 g de phénylsulfure acétal $C_{20}$ diméthylique (7E, 9E, 13E) et 3,95 g d'un mélange de phénylsulfure acétal $C_{20}$ diméthylique (7E, 9E, 13E ; 7E, 9E, 13Z ; 7E, 9Z, 13E et 7E, 9Z, 13Z).

Le rendement est voisin de 85 %.

2) A une suspension de 3 g de silice (silice MERCK) dans 5 cm3 de chlorure de méthylène, on ajoute 0,3 cm3 d'une solution aqueuse d'acide oxalique à 10 % puis, à l'abri de la lumière et sous atmosphère d'argon, 1 g de phénylsulfure acétal $C_{20}$ diméthylique.

Après 15 minutes à une température voisine de 20°C, la réaction est terminée. On dilue par addition de 3 cm3 de chlorure de méthylène puis filtre sur verre fritté n° 4 sous atmosphère d'azote. On lave la silice par 3 fois 5 cm3 de chlorure de méthylène. Les phases organiques sont séchées sur sulfate de sodium. Après filtration et évaporation du solvant sous pression réduite à 15°C, on obtient 0,855 g de phénylsulfure aldéhyde $C_{20}$.

Le rendement est de 95 %.

3) A 0,788 g de phénylsulfure aldéhyde $C_{20}$ en solution dans 2 cm3 d'éthanol absolu, on ajoute à 20°C, sous atmosphère d'argon et à l'abri de la lumière, 2 cm3 d'une solution 1N d'éthylate de sodium dans l'éthanol. Après 15 minutes, on ajoute 10 cm3 d'eau et 10 cm3 de chlorure de méthylène. Après décantation, la phase organique est lavée avec une solution saturée de chlorure de sodium jusqu'à pH = 7 puis est séchée sur sulfate de sodium. Après filtration et évaporation du solvant sous atmosphère d'argon et à l'abri de la lumière on obtient 547 mg d'une huile qui est constituée de 70 % de rétinal tout trans, de 8 % d'isomère 9-cis, de 20 % d'isomère 13-cis et de 2 % d'isomère dicis.

Le rendement est de 96 %.

## Revendications

1. Procédé de préparation du rétinal caractérisé en ce que l'on condense un sulfure $C_{15}$ de formule générale :

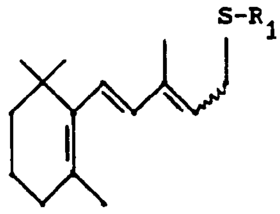

dans laquelle $R_1$ représente un radical aryle, aralkyle, pyridyle, thiazolyl-2, benzothiazolyl-2, benzimidazolyl-2 ou benzoxazolyl-2, sur un halogénoacétal de formule générale :

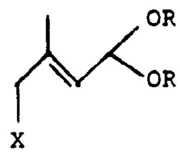

dans laquelle X représente un atome d'halogène et R représente un radical alcoyle contenant 1 à 4 atomes de carbone puis hydrolyse et désulfure le sulfure acétal $C_{20}$ de formule générale :

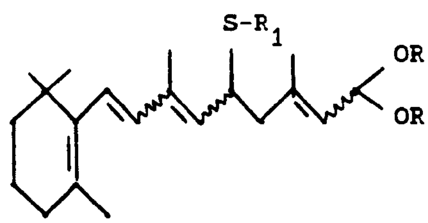

dans laquelle $R_1$ et R sont définis comme précédemment, en passant intermédiairement par le sulfure aldéhyde $C_{20}$ de formule générale :

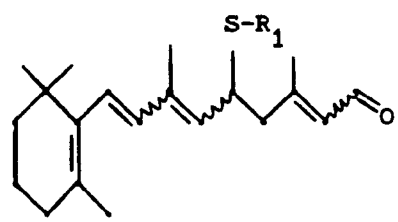

dans laquelle $R_1$ est défini comme précédemment, et isole le rétinal ainsi obtenu.

2. Procédé selon la revendication 1 caractérisé en ce que la condensation du sulfure $C_{15}$ sur l'halogénoacétal $C_5$ est effectuée en présence d'un agent basique, éventuellement en association avec un second agent basique destiné à neutraliser l'hydracide formé, en opérant dans un solvant organique à une température comprise entre -100 et 100°C.

3. Procédé selon la revendication 2 caractérisé en ce que l'agent basique est choisi parmi les hydroxydes, alcoolates, hydrures ou amidures de métaux alcalins et les dérivés organozinciques, organolithiens ou organomagnésiens.

4. Procédé selon la revendication 2 caractérisé en ce que le solvant est choisi parmi les hydrocarbures aliphatiques, les hydrocarbures aromatiques, les alcools, les éthers, les amides, le diméthylsulfoxyde, la N-méthylpyrrolidone ou l'hexaméthylphosphotriamide.

5. Procédé selon la revendication 1 caractérisé en ce que l'hydrolyse du sulfure acétal $C_{20}$ est effectuée en milieu acide.

6. Procédé selon la revendication 1 caractérisé en ce que la désulfuration du sulfure aldéhyde $C_{20}$ est effec-

tuée au moyen d'un agent basique minéral ou organique choisi parmi les hydroxydes, les carbonates ou les alcoolates alcalins.

7. Le sulfure acétal $C_{20}$ de formule générale :

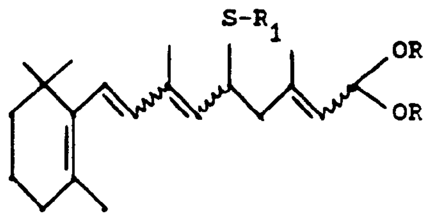

dans laquelle $R_1$ représente un radical aryle, aralkyle, pyridyle, thiazolyl-2, benzothiazolyl-2, benzimidazolyl-2 ou benzoxazolyl-2 et R représente un radical alcoyle contenant 1 à 4 atomes de carbone.

8. Le sulfure aldéhyde $C_{20}$ de formule générale :

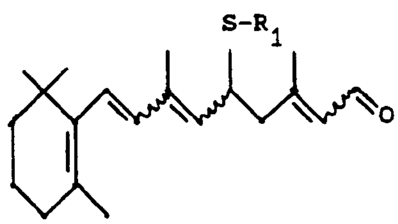

dans laquelle $R_1$ représente un radical aryle, aralkyle, pyridyle, thiazolyl-2, benzothiazolyl-2, benzimidazolyl-2 ou benzoxazolyl-2.

**Patentansprüche**

1. Verfahren zur Herstellung von Retinal, dadurch gekennzeichnet, daß man ein $C_{15}$-Sulfid der allgemeinen Formel:

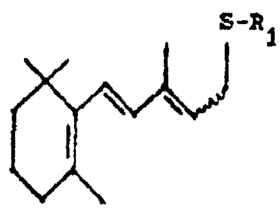

in welcher $R_1$ einen Aryl-, Aralkyl-, Pyridyl-, 2-Thiazolyl-, 2-Benzothiazolyl-, 2-Benzimidazolyl- oder 2-Benzoxazolylrest darstellt, mit einem Halogenacetal der allgemeinen Formel:

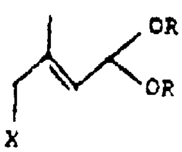

in welcher X ein Halogenatom darstellt und R einen Alkylrest mit 1 bis 4 Kohlenstoffatomen darstellt, kondensiert, dann das $C_{20}$-Acetalsulfid der allgemeinen Formel:

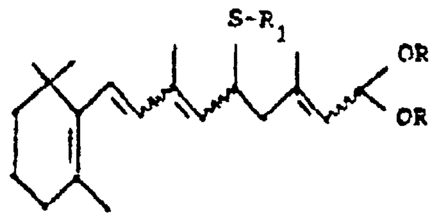

in welcher $R_1$ und R die obige Bedeutung haben, entschwefelt, wobei das $C_{20}$-Aldehydsulfid der allgemeinen Formel:

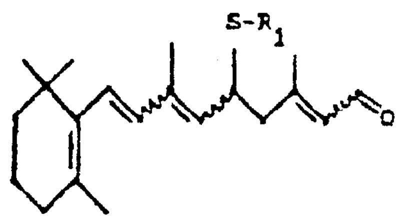

in welcher $R_1$ die obige Bedeutung hat, als Zwischenprodukt durchlaufen wird, und das so erhaltene Ratinal isoliert.

2. Verfahren nach Anspruch 1, dadurch gekennzeichnet, daß die Kondensation des $C_{15}$-Sulfids mit dem $C_5$-Halogenacetal in Gegenwart eines basischen Mittels, gegebenenfalls kombiniert mit einem zweiten basischen Mittel, das die gebildete Wasserstoffsäure neutralisieren soll, ausgeführt wird, indem man in einem organischen Lösungsmittel bei einer Temperatur zwischen -100 und 100°C arbeitet.

3. Verfahren nach Anspruch 2, dadurch gekennzeichnet, daß das basische Mittel ausgewählt wird aus den Alkalimetall- hydroxiden, -alkoholaten, -hydriden oder -amiden und den Organozinn-, Organolithium- oder Organomagnesiumverbindungen.

4. Verfahren nach Anspruch 2, dadurch gekennzeichnet, daß das Lösungsmittel ausgewählt wird aus den aliphatischen Kohlenwasserstoffen, den aromatischen Kohlenwasserstoffen, den Alkoholen, den Äthern, den Amiden, Dimethylsulfoxid, N-Methylpyrrolidon oder Hexamethylphosphotriamid.

5. Verfahren nach Anspruch 1, dadurch gekennzeichnet, daß die Hydrolyse des $C_{20}$-Acetalsulfids in saurem Milieu ausgeführt wird.

6. Verfahren nach Anspruch 1, dadurch gekennzeichnet, daß die Entschwefelung des $C_{20}$-Aldehydsulfids mittels eines anorganischen oder organischen Mittels, ausgewählt aus den Alkali- hydroxiden, -carbonaten oder -alkoholaten, ausgeführt wird.

7. $C_{20}$-Acetalsulfid der allgemeinen Formel:

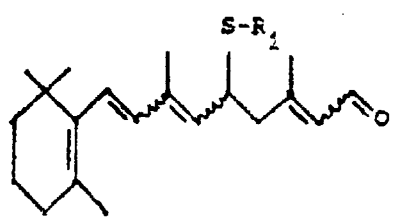

in welcher $R_1$ einen Aryl-, Aralkyl-, Pyridyl-, 2-Thiazolyl-, 2-Benzothiazolyl-, 2-Benzimidazolyl- oder 2-Benzoxazolylrest und R einen Alkylrest mit 1 bis 5 Kohlenstoffatomen darstellt.

8. $C_{20}$-Aldehydsulfid der allgemeinen Formel:

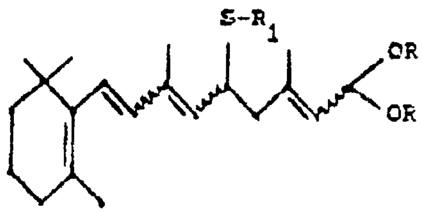

in welcher $R_1$ einen Aryl-, Aralkyl-, Pyridyl-, 2-Thiazolyl-, 2-Benzothiazolyl-, 2-Benzimidazolyl- oder 2-Benzoxazolylrest darstellt.

**Claims**

1. Process for preparing retinal, characterised in that a $C_{15}$ sulphide of general formula:

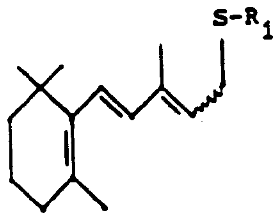

in which $R_1$ denotes an aryl, aralkyl, pyridyl, 2-thiazolyl, 2-benzothiazolyl, 2-benzimidazolyl or 2-benzoxazolyl radical, is condensed with a haloacetal of general formula:

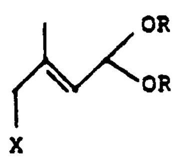

in which X denotes a halogen atom and R denotes an alkyl radical containing 1 to 4 carbon atoms, the $C_{20}$ sulphide acetal of general formula:

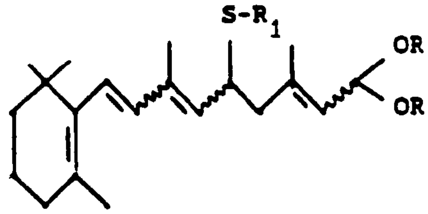

in which $R_1$ and R are defined as above, is then hydrolysed and desulphurised, proceeding via the intermediate $C_{20}$ sulphide aldehyde of general formula:

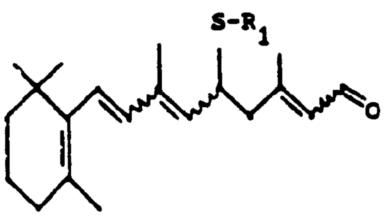

in which $R_1$ is defined as above, and the retinal thereby obtained is isolated.

2. Process according to Claim 1, characterised in that the condensation of the $C_{15}$ sulphide with the $C_5$ haloacetal is performed in the presence of a basic agent, optionally in combination with a second basic agent whose purpose is to neutralise the hydracid formed, working in an organic solvent at a temperature between -100 and 100°C.

3. Process according to Claim 2, characterised in that the basic agent is chosen from alkali metal hydroxides, alcoholates, hydrides or amides and organozinc, organolithium or organomagnesium derivatives.

4. Process according to Claim 2, characterised in that the solvent is chosen from aliphatic hydrocarbons, aromatic hydrocarbons, alcohols, ethers, amides, dimethyl sulphoxide, N-methylpyrrolidone or hexamethylphosphotriamide.

5. Process according to Claim 1, characterised in that the hydrolysis of $C_{20}$ sulphide acetal is performed in an acid medium.

6. Process according to Claim 1, characterised in that the desulphurisation of the $C_{20}$ sulphide aldehyde is accomplished by means of an inorganic or organic basic agent chosen from alkali metal hydroxides, carbonates or alcoholates.

7. The $C_{20}$ sulphide acetal of general formula:

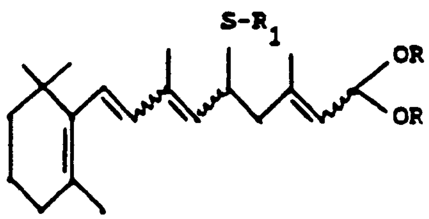

in which $R_1$ denotes an aryl, aralkyl, pyridyl, 2-thiazolyl, 2-benzothiazolyl, 2-benzimidazolyl or 2-benzoxazolyl radical and R denotes an alkyl radical containing 1 to 4 carbon atoms.

8. The $C_{20}$ sulphide aldehyde of general formula:

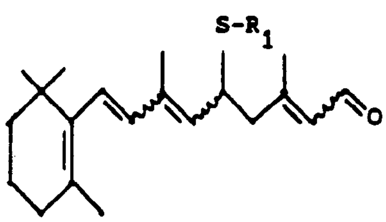

in which $R_1$ denotes an aryl, aralkyl, pyridyl, 2-thiazolyl, 2-benzothiazolyl, 2-benzimidazolyl or 2-benzoxazolyl radical.